# EUROPEAN PATENT APPLICATION

(11) **EP 3 296 292 A1**
(43) Date of publication of application: **21.03.2018**
(21) Application number: 16189458.9
(22) Date of filing: 19.09.2016
(51) Int. Cl.: C07D 211/60

(54) **METHOD FOR THE PREPARATION OF ALKYL PHENIDATES**

(71) Applicant: Université de Liège, 4000 Liège (BE)
(72) Inventor: MONBALIU, Jean-Christophe, B-4000 Liège (BE); GERARDY, Romaric, B-4000 Liège (BE)
(74) Representative: Peel, James Peter

(57) **Abstract**

The invention provides a method for the preparation of an intermediate for use in synthesizing a lower alkyl phenidate compound of formula (I): 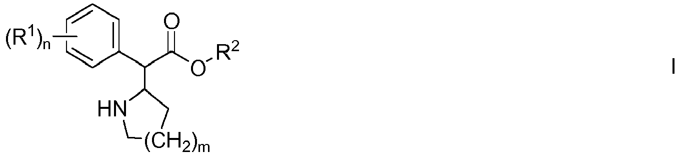 wherein each R¹ independently represents an optionally substituted aryl, heteroaryl, alkyl, cycloalkyl, alkoxy, aryloxy, acyl, carboxyl, hydroxyl, halogen, amino, nitro, sulfo or sulfhydryl group, R² represents a hydrogen atom or a lower alkyl group, n represents an integer from 1 to 5 and m represents an integer from 1 to 3 or a pharmaceutically acceptable salt thereof;
which method comprises the steps of:
(a) flowing a tosylhydrazone compound of formula (IV): 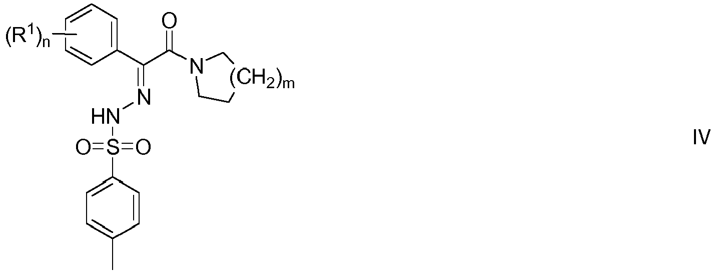 wherein R¹, n and m are as defined above in relation to the methylphenidate of formula (I), an organic base and an organic solvent into a fluidic network; and
(b) reacting the tosylhydrazone compound of formula (IV) and the base in the fluidic network under thermal and/or photochemical conditions to form a transient diazoamide compound of formula (V): 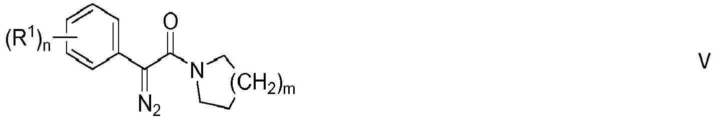 wherein R¹, n and m are as defined above in relation to the methylphenidate of formula (I).

## Description

The present invention relates to an improved process for the preparation of a lower alkyl phenidate and a prodrug or intermediate thereof, using a continuous-flow process.

A significant lower alkyl phenidate is methylphenidate which is referred to herein as the compound of formula (1):

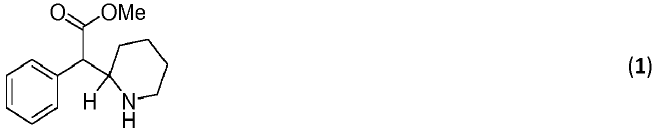

Methylphenidate is a central nervous system stimulant and is the most widely prescribed stimulant medication to treat attention deficit hyperactivity disorder (ADHD) and narcolepsy. Its global consumption has dramatically increased since the early 1990's. The pharmacodynamics of methylphenidate is similar to cocaine: it binds and blocks the dopamine and norepinephrine transporters, therefore acting as a norepinephrine-dopamine reuptake inhibitor, which results in higher synaptic levels of both neurotransmitters.

Low doses of methylphenidate activate both dopamine and norepinephrine neurotransmission within the prefrontal cortex. Following oral administration, maximal blood concentration is reached after *ca*. 2 h, and 90% is excreted in urine after 48 h (50% after 8 h). Urine analysis of treated patients demonstrated that the main methylphenidate metabolite is ritalinic acid. Methylphenidate is generally well-tolerated, and less prone to abuse than other central nervous system stimulants. Mild adverse effects upon prolonged treatment such as nervousness, insomnia, hypersensitivity, decreasing appetite, nausea, palpitations, hypertension, tachycardia, dizziness, drowsiness, headaches and diskynesia have been reported, but are thought to be associated to a mild overdosage.

Methylphenidate (also known as methyl 2-phenyl-2-(piperidin-2-yl)acetate) has two stereogenic centers that account for 4 stereoisomers, namely, (2R,2'R)-threo (referred to herein as the compound of formula (**1a**)), (2S,2'S)-threo (referred to herein as the compound of formula (**1b**))**,** (2R,2'S)-erythro (referred to herein as the compound of formula (**1c**)) and (2S,2'R)-erythro methylphenidate (referred to herein as the compound of formula (**1d**)):

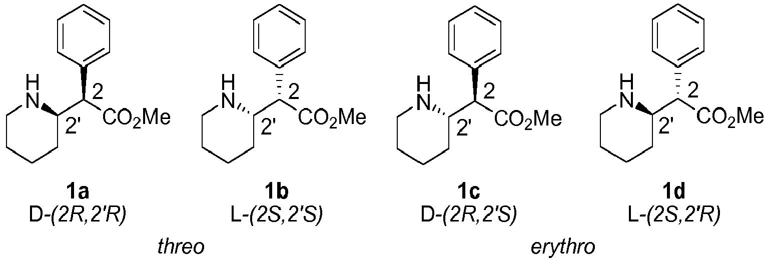

The threo racemate (which comprises a mixture of the compounds of formula (**1a**) and (**1b**)) is responsible for the stimulant activity, with the (2R,2'R)-threo enantiomer being 5 to 38 times more active that the (2S,2'S)-threo enantiomer (K. S. Patrick et al., 1987, J. Pharmacol. Exp. Ther. 241:152-158). The single (2R,2'R)-threo enantiomer, also named dexmethylphenidate, is commercialized under the trade name Focalin^{®}, while the threo racemic mixture is sold under various names such as Ritalin^{®} or Concerta^{®}. Both forms are prescribed to treat ADHD. The active pharmaceutical ingredient methylphenidate is usually formulated as a hydrochloride salt.

A variety of methods have been reported for the preparation of methylphenidate. Most of these methods afforded stereoselectively the *threo* racemate, and some methods were dedicated to the enantioselective preparation of (2R,2'R)-threo methylphenidate. Some methods have been reported in the art with expensive metal catalysts with combined yields ranging from 64 to 73%, enantiomeric excesses ranging from 69 to 86% and diastereomeric excesses ranging from 42 to 94%. These methods come with shortcomings associated to trace contamination of the final product with toxic metals and poor global efficiency.

Metal-free methods have been reported, as for instance the methods described by Winkler (WO99/36403), which was later slightly modified by Gutman (WO2004/080583). These methods were based on a sequence of reactions, as follows (wherein Ts refers to a tosyl group):

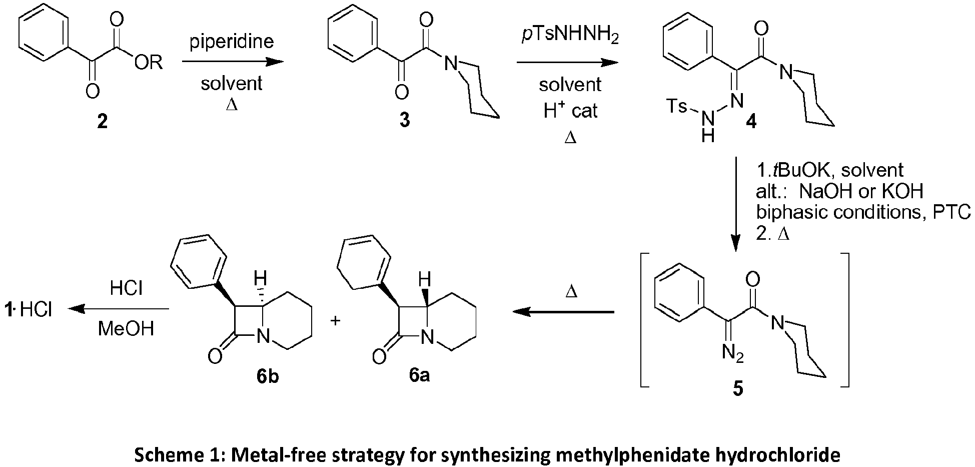

The starting material is an alkyl phenylglyoxalate (referred to herein as the compound of formula (**2**)). The compound of formula **(2)** was condensed with piperidine at temperatures ranging from 45 to 100 °C, in the presence of a solvent such as methanol, affording 1-phenylglyoxylylpiperidine (referred to herein as the compound of formula **(3)**) in high yields (75-96%). In the next step, the compound of formula **(3)** was reacted with *p*-toluenesulfonyl hydrazide in the presence of an acid catalyst such as sulfuric or hydrochloric acid in a solvent such as ethanol or dioxane at reflux for up to 7 h, giving up to 91% yield of the corresponding tosylhydrazone (referred to herein as the compound of formula **(4).** Next, refluxing a solution of the compound of formula **(4)** for a minimum of 90 minutes in toluene in the presence of an inorganic base such as an alkali hydroxide (according to WO2004/080583) or alkoxide (according to WO 99/36403 and Axten et al., 1998, J. Org. Chem. 63:9628-9629) to trigger the thermal decomposition of the compound of formula **(4)** into the corresponding transient diazo (referred to herein as the compound of formula **(5),** which was subsequently transformed into a β-lactam intermediate (referred to herein as the compound of formula **(6)** in moderate to high yield. In case an alkali hydroxide is used, biphasic conditions were used in the presence of a phase transfer catalysis (PTC) to vehicle the inorganic base from the aqueous phase to the organic phase, and longer reaction times up to 6.5 h were reported (WO2004/080583). The main feature of interest in these procedures resides in the stereoselective formation of the threo β-lactam (referred to herein as the compound of formula **(6)** wherein the threo stereoisomer is referred to as being the compound of formula **(6a)** and the erythro stereoisomer is referred to as being the compound of formula **(6b)** and wherein the molar ratio of the threo **(6a)** to the erythro **(6b)** is from 3.3:1 to 6:1). β-lactam of formula **(6)** can also be obtained from the photochemical decomposition of the same tosylhydrazone of formula **(4)** and of the transient diazo of formula **(5),** but only in low yield (Hurst, 1969, J. Chem. Soc (C) 2093-2098).

Finally, methylphenidate hydrochloride of formula (**1**)·HCl was obtained after acidic methanolysis of β-lactam of formula **(6),** with retention of stereochemistry. Other methods were reported for the resolution of the threo racemate of methylphenidate by formation of diastereomeric salt involving chiral resolution reagents (US Patents No 6,162,919 and 6,100,401). Zeitlin also reported the use of enzymes for producing chiral compounds, including methylphenidate, from β-lactam intermediate of formula **(6)** (US Patent No 5,733,756).

Since these methods are based on stepwise macroscopic batch processes, they come with various shortcomings inherent to the technology, such as poor mixing and heat transfer, which ultimately account for low efficiency and productivity, quality deficiency and poor flexibility. Besides, an increasing chemical risk is associated with classical large scale, stepwise batch processes, in particular for multistep sequences involving highly reactive species or hazardous/explosive intermediates. In particular, the handling and the decomposition of transient diazo species such as compound of formula **(5)** under thermal conditions on industrial scale significantly rise the chemical hazard associated with the process. Additionally, the alternate photochemical decomposition of the compound of formula **(5)** on a preparative scale will be hampered by the poor penetration of light in a batch setup, hence leading to prolonged irradiation times, leading to overexposure, side reactions and an increase of the processing cost. The biphasic systems described in the art come also with non-negligible amounts of side product due to the insertion of water in the transient carbene generated after decomposition of the diazo of formula **(5).**

A way of ameliorating these problems has been sought.

### Description of the invention

According to the invention there is provided a method for the preparation of an intermediate for use in synthesizing a lower alkyl phenidate compound of formula (I): 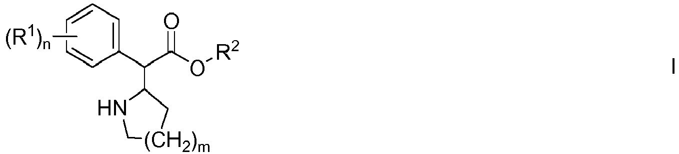 wherein each R¹ independently represents an optionally substituted aryl, heteroaryl, alkyl, perfluoroalkyl, cycloalkyl, alkoxy, aryloxy, acyl, carboxyl, hydroxyl, halogen, amino, nitro, cyano, sulfo or sulfhydryl group, R² represents a hydrogen atom or a lower alkyl group, n represents an integer from 1 to 5 and m represents an integer from 1 to 3 or a pharmaceutically acceptable salt thereof;
which method comprises the steps of: (a) flowing a tosylhydrazone compound of formula (IV): 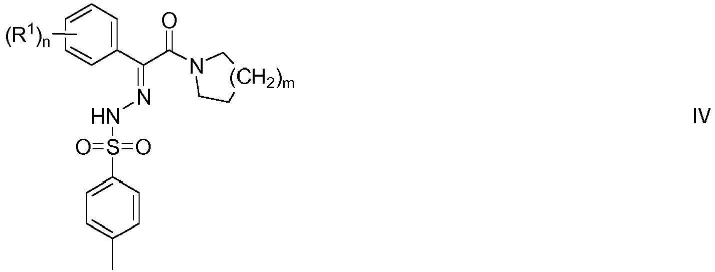 wherein R¹, n and m are as defined above in relation to the lower alkyl phenidate of formula (I), an organic base and an organic solvent into a fluidic network; and
(b) reacting the tosylhydrazone compound of formula (IV) and the base in the fluidic network under thermal and/or photochemical conditions to form a transient diazoamide compound of formula (V): 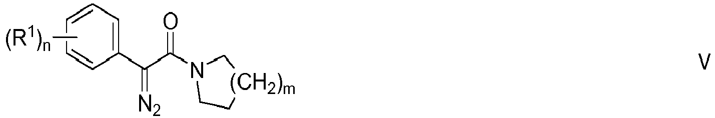 wherein R¹, n and m are as defined above in relation to the lower alkyl phenidate of formula (I).

The present invention overcomes the shortcomings of the batch processes, and provides a scalable, safe and intensified continuous-flow process towards a lower alkyl phenidate or a prodrug or a derivative or an intermediate thereof with a minimal footprint. The present invention provides various methods for synthesizing a pharmaceutically active species, or an intermediate thereof, by using thermal or photochemical continuous-flow conditions with a drastically improved efficiency by comparison to the batch procedures. The process enables shorter reaction times than for the batch processes (from several hours in batch to a few minutes in a flow process). The fluidic network setup may use intensified thermal conditions to accelerate the chemical processes and reactions, and at least one critical step can be alternatively performed under photochemical conditions.

The method enables an accurate control on the reaction conditions, such as a control of the temperature, the pressure, the flow rate and the local stoichiometry. The method also permits to obtain a product with a constant quality and purity profile. The method enables a substantially safer handling of transient or explosive chemicals such as diazo species.

This method also provides a continuous-flow method for synthesizing a pharmaceutically active species, including flowing a fluid sample comprising a pharmaceutically active species precursor such as a β-lactam of formula (VI) into a micro-/meso-channel; and/or performing an in-line purification of the pharmaceutically active species precursor; and/or performing an in-line analysis of the pharmaceutically active species precursor; and/or performing a chemical reaction, in the micro-/meso-channel, to convert the pharmaceutically active species precursor to the pharmaceutically active species or intermediate thereof. By streamlining multiple operations within the same uninterrupted fluidic reactor network comprising micro-/meso-channels, the present invention thus alleviates the handling of any of the chemical intermediates and large inventories of chemical intermediates.

The continuous-flow micro-/meso-reactor network is compact and may be used as a mobile and/or a versatile production platform. This microfluidic process is straightforward, versatile, economic, safe, and provides high yields (up to 99% conversion) and excellent molecular control for the production of active pharmaceutical ingredients, such as methylphenidate hydrochloride. Most importantly, the fluidic setup reduces the global footprint and the workforce required for producing large inventories of active pharmaceutical ingredients, such as methylphenidate hydrochloride. The process is amenable to the production of libraries of methylphenidate derivatives using diverse aromatic substitution and diverse nitrogen-containing heterocycles. For example methylphenidate derivatives disclosed in US 8,076,485 are incorporated by reference. The process further enables a productivity that can be adapted depending on the demand.

In some embodiments, the method comprises reacting a tosylhdrazone compound of formula (IV) and an organic base for a reaction time of about 60 min or less, to produce a transient diazoamide species of formula (V). In some embodiments, a fluidic network comprising micro- and/or meso-channels is utilized. Micro-channel means a channel with an internal dimension ranging from 100 µm to 850 µm. A micro-fluidic reactor comprises a network of micro-channels and various fluidic elements assembled in modules. Meso-channel means a channel with an internal dimension ranging from 850 µm to 2000 µm. A meso-fluidic reactor comprises a network of meso-channels and various fluidic elements assembled in modules.

In some embodiments, the method according to the invention comprises an additional step:
(c) thermal and/or photochemical decomposition of the diazoamide compound of formula (V) in a fluidic network to yield a pharmaceutically active species precursor such as a β-lactam of formula (VI): 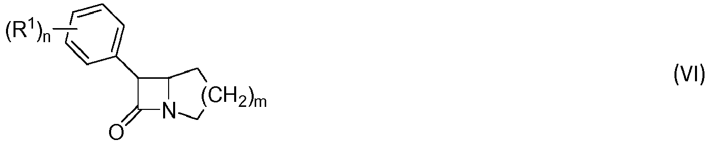 wherein R¹, m and n are as defined above in relation to the lower alkyl phenidate of formula (I).

In some embodiments, step (c) comprises a C-H carbene insertion. In some embodiments, step (c) may have a yield of more than 50%. In some embodiments, step (c) uses a fluidic network comprising micro- and/or meso-channels.

In some embodiments, step (c) can be telescoped to subsequent process operations, such that step (c) additionally comprises a step of an in- or off-line downstream purification including quench, liquid-liquid extraction, liquid-liquid separation, gas-liquid separation, filtration on silica gel or recrystallization. In some embodiments, liquid-liquid extraction requires the injection of a secondary phase, such as an aqueous phase. In some embodiments, the aqueous phase contains an inorganic salt such as sodium chloride. In some embodiments, in-line liquid-liquid or gas-liquid separation involves a membrane or a settling tank. In some embodiments, the liquid-liquid separation and the gas-liquid separation are effected at the same time.

In some embodiments, step (c) can be telescoped to subsequent chemical operations, including chemical reactions, such that step (c) additionally one of the following reactions:
(i) a reaction of the β-lactam of formula (VI) with a lower alkyl alcohol in the presence of a pharmaceutically acceptable acid catalyst to form a lower alkyl phenidate of formula (I) or a salt or an analog thereof; or
(ii) a reaction with an organic and/or inorganic base or acid to increase the amount of a specific stereoisomer of the compound of formula (VI), such as the threo racemate; or
(iii) a reaction with an organic and/or inorganic base or acids to increase the amount of a specific stereoisomer of the compound of formula (I), such as the threo racemate.

In some embodiments, step (c) (i) of the method is performed for a reaction time of about 30 min or less. In some embodiments, steps (c) (ii) and (c) (iii) of the method of the invention are performed for a reaction time of about 60 min or less. Reaction time may alternatively be expressed as a residence time in the fluidic network. Residence time means the actual process time under the specific process conditions (temperature, pressure, irradiation) inside the controlled environment of a micro-/meso-fluidic reactor and is generally expressed in minutes. The residence time is calculated by the ratio of the internal volume of the fluidic reactor and the total flow rate in the fluidic reactor. Telescoping means the integration of multiple process or chemical operations within the same uninterrupted fluidic reactor network. In some embodiments, the method of the invention comprises in-line analysis including but not restricted to in-line IR monitoring or other spectroscopic methods for process monitoring purposes.

In some embodiments, in step (c) (i) the generation of pharmaceutically active species precursor such as a β-lactam 6 is telescoped with a reaction or a sequence of reactions that involves hydrochloric acid in the presence of a lower alkyl alcohol. In some embodiments, the reaction mixture involves a lower alkyl alcohol and an ether such as a dialkyl ether or an oxygenous heterocycle such as, but not restricted to, tetrahydrofuran or 1,4-dioaxane, in a ratio by volume ranging from 1:1 to 1:10, for example from 1:1 to 1:3. In some embodiments, the reaction conditions involve molar ratios between the β-lactam of formula (VI) and the pharmaceutically acceptable acid ranging from 1:1 to 1:10, for example from 1:1 to 1:3. In some embodiments, step (c) (i) comprises the reaction or the sequence of reactions of a β-lactam of formula (VI) with a mixture of solvents comprising methanol and an ether in the presence of the pharmaceutically acceptable acid (for example HCl) to yield enriched threo lower alkyl phenidate acid salt (for example hydrochloride) in 99% conversion. Threo lower alkyl phenidate acid salt is a 50:50 molar mixture of compounds of formula (IA) and (IB), as follows: 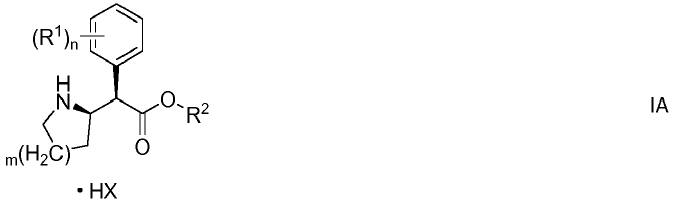 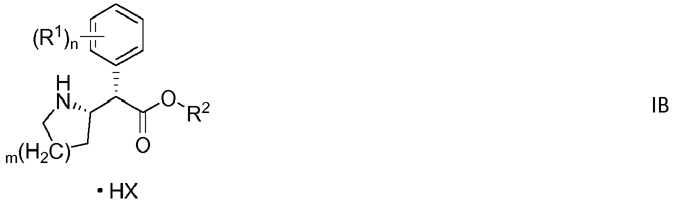 wherein R¹, R², n and m are as defined above for the compound of formula (I), and where HX is the pharmaceutically acceptable acid.

In some embodiments, the fully telescoped system gave enriched threo pharmaceutically acceptable acid salt of lower alkyl phenidate of formula (IA and IB) in 70% isolated yield.

The tosylhydrazone of formula (IV) which is the starting material of the method of the invention is available following an adapted procedure from the art (Axten et al., 1998, J. Org. Chem. 63:9628-9629). In some embodiments, the tosylhydrazone of formula (IV) is prepared under a continuous-flow strategy using a flow reactor comprising micro- and/or meso-fluidic modules. In some embodiments, the tosylhydrazone of formula (IV) is obtained under continuous-flow conditions by adapting the known batch procedures. In some embodiments, the conditions may require heating solids above their melting points.

In some embodiments, the organic base used in the method of the invention may be a nitrogenous organic base. A suitable nitrogenous organic base is a trialkylamine such as trimethylamine or diisopropylethylamine, tetramethylguanidine and other guanidine derivatives, 1,8-diazabicycloundec-7-ene (DBU) and other nitrogenous heterocyclic bases, or phosphazene-type bases that have the appropriate pKa to deprotonate the hydrazone and that confers to the corresponding ionic compound a good solubility in an organic solvent such as toluene, *N-*methylpyrrolidinone, propylene carbonate or acetonitrile.

In some embodiments, the method of the invention uses an aprotic polar solvent or a non-water soluble apolar aprotic organic solvent such as toluene. In some embodiments, step (a) may include flowing a phase transfer catalyst into the fluidic network. In some embodiments, the phase transfer catalyst may be a quaternary ammonium salt. A suitable quaternary ammonium salt is tetrabutylammonium chloride, or trioctylmethylammonium chloride. In some embodiments, the phase transfer catalyst may be used in an amount from 0.01 mol% to 50 mol%, for example from 1 mol% to 15 mol% with reference to the limiting reactant. In some embodiments, the presence of the phase transfer catalyst may improve the solubility of the tosylhydrazone of formula (IV) in a non-polar non-water miscible organic solvent such as toluene, or/and may improve the solubility of the salt derived from tosylhydrazone of formula (IV) in a non-polar non-water miscible organic solvent such as toluene. An increased solubility means that no precipitate is formed at a concentration of at least 0.1 mol L⁻¹ in a suitable solvent, for example at a concentration of 0.75 mol L⁻¹. An increased solubility alternatively means that no precipitate is formed even after several hours, for example after 5 hours.

In some embodiments, the method of the invention using thermal decomposition yields β-lactam of formula (VI) with a threo/erythro ratio ranging from 3:1 to 5:1; for example with a conversion of up to 99%.

In some embodiments, the method of the invention using photochemical decomposition yields β-lactam of formula (VI)with a threo/erythro ratio ranging from 4:1 to 5:1; for example with a conversion of up to 97%.

Herein "acyl" means a moiety of formula -C(O)-R³ where R³ represents a hydrogen atom or an alkyl, cycloalkyl or aryl group. "Amino" means a moiety of formula -NR⁴R⁵ where each of R⁴ and R⁵ independently represents a hydrogen atom or a lower alkyl group. "Alkoxy" means a moiety of formula -OR⁶ where R⁶ represents an alkyl group. In some embodiments, an alkoxy group may be a methoxy group (-OCH₃). "Alkyl" means a monovalent optionally substituted saturated straight- or branched- chain hydrocarbon group containing from 1 to 8 carbon atoms. In some embodiments, each alkyl group may, optionally, be substituted with one or more amino, hydroxyl or sulfhydryl groups. "Perfluoroalkyl" means a monovalent optionally substituted saturated straight- or branched- chain alkyl group containing from 1 to 8 carbon atoms in which every hydrogen has been replaced by a fluorine. "Aryl" means a monovalent optionally substituted mono-, bi- or tricyclic aromatic hydrocarbon group having from 6 to 14 ring carbon atoms; for example a phenyl group. "Aryloxy" means a group of formula -OR⁷ wherein R⁷ represents an aryl group. In some embodiments, an alkoxy group may be phenoxy. "Carboxyl" means a moiety of formula -C(O)OR³ wherein R³ represents a hydrogen atom or an alkyl, cycloalkyl or aryl group. "Cycloalkyl" means a saturated optionally substituted monovalent mono- or bicyclic hydrocarbon moiety of from 3 to 10 ring carbon atoms. In some embodiments, the cycloalkyl moiety contains from 4 to 8 ring carbon atoms, for example cycloalkyl may be cyclohexyl. "Halogen" means a chlorine, fluorine, bromine or iodine atom. In some embodiments, halogen may be a chlorine or bromine atom. "Heteroaryl" means a monovalent monocyclic or bicyclic aromatic moiety of from 5 to 12 ring atoms containing one, two, or three ring heteroatoms each of which is independently selected from a nitrogen, oxygen, and/or Sulphur atom, where the remaining ring atoms are each a carbon atom. "Hydroxyl" means a -OH moiety. "Lower alkyl" means a saturated optionally substituted straight- or branched- chain hydrocarbon containing from 1 to 4 carbon atoms. In some embodiments, lower alkyl means a methyl moiety. "Nitro" means a -NO₂ group. "Cyano" means a -CN group. "Sulfhydryl" means a -SH group. "Sulfo" means a -SO₃H group. Ts means tosyl or a CH₃C₆H₄SO₂- group."Prodrug" means any compound which releases a lower alkyl phenidate of formula (I) *in vivo* when such prodrug is administered to a mammal. In some embodiments, a prodrug of a compound of formula (I) may be prepared by modifying one or more functional group(s) present in the compound of formula (I) such that the modified functional group(s) may be cleaved *in vivo* to release the corresponding compound of formula (I). For example, a prodrug may include a compound of formula (I) where a hydroxy, amino, or sulfhydryl group is bonded to any group that may be cleaved *in vivo* to generate the free hydroxyl, amino, or sulfhydryl group, respectively. Examples of prodrugs include an ester or carbamate of a hydroxy functional group in a compound of formula (I). "Organic base" means any nitrogenous organic compound with basic properties such as, but not restricted to, triethylamine, N,N-diisopropylethylamine, tetramethylguanidine, 1,8-diazabicycloundec-7-ene. "Organic acid" means any organic compound with acidic properties such as, but not restricted to, such as acetic, propionic, succinic and other carboxylic acids. "Inorganic base" means alkali or alkaline earth metal salts such as, but not restricted to, hydroxide, phosphate and carbonate salts. "Inorganic acid" means mineral acid compounds such as hydrochloric, hydrobromic, sulfuric, phosphoric, nitric acid. "Pharmaceutically acceptable salt" means a conventional non-toxic salts, such as a salt derived from an inorganic acid (such as hydrochloric, hydrobromic, sulfuric, phosphoric, nitric acid), an organic acids (such as acetic, propionic, succinic, glycolic, stearic, lactic, malic, tartaric, citric, glutamic, aspartic, benzoic, salicylic, oxalic, ascorbic acid). A pharmaceutically acceptable salt may be prepared in a conventional manner, e.g., by reacting the free base form of the compound of formula (I) with a suitable acid.

In some embodiments, the method of the invention uses a fluidic network to generate intensified continuous-flow thermal conditions for synthesizing a pharmaceutically active species (such as a compound of formula (I)), or a pharmaceutically active species precursor thereof (such as a compound of formula (V) or a compound of formula (VI)) with a short reaction time such as a reaction time less than an hour, such as less than 30 minutes or less than 5 min by utilizing a temperature from room temperature to 200 °C, for example from 45 °C to 180 °C, and a counter pressure ranging from 1 to 100 bar, preferably from 2 to 20 bar. In some embodiments, the present invention uses intensified continuous-flow photochemical conditions for synthesizing a pharmaceutically active species precursor (such as a compound of formula (V) or a compound of formula (VI)) using a short reaction time such as a reaction time less than 2 hours, such as a reaction time less than 60 min by utilizing a high output light source emitting in the 275 to 450 nm wavelength range, for example from 350 to 430 nm, and more specifically from 365 to 405 nm and a temperature from room temperature to 100 °C, for example from 20 °C to 45 °C, and a counter pressure from 1 to 100 bar, for example from 2 to 20 bar. The present invention also discloses the integration of multiple steps, including chemical and process steps for synthesizing a pharmaceutically active species (such as a compound of formula (I)), or pharmaceutically active species precursor thereof (such as a compound of formula (V) or a compound of formula (VI)). Process parameters include a flow rate ranging from 1 µL min⁻¹ to 1000 mL min⁻¹, optionally ranging from 100 µL min⁻¹ to 100 mL min⁻¹, and more specifically from 10 µL min⁻¹ to 30 mL min⁻¹.

In some embodiments, the fluidic network comprises one or more fluidic modules, each associated with a specific process step such as, but not restricted to, in-line purification, in-line analysis and chemical transformations. In some embodiments, the fluidic modules may be constructed from a material with a high tolerance to a wide range of chemicals and a good tolerance to high temperature and pressure, such as glass, specialty glass, quartz, fused silica, or perfluorinated polymers such as PFA, ETFE or PTFE. In some embodiments, the fluidic network comprises micro-fluidic modules constructed from a coil of a perfluorinated polymer with defined sections and volumes. In some embodiments, the fluidic network reactor comprises meso-fluidic modules constructed from specialty glass with defined sections and volumes. In some embodiments, the fluidic modules are constructed from a transparent material, such as glass, specialty glass such as Pyrex glass, quartz or fused silica, or perfluorinated polymers such as PFA, ETFE or PTFE. In some embodiments, two or more fluidic networks may be operated in series and/or in parallel; the two or more fluidic networks may be substantially identical.

The invention is illustrated with reference to the following Figures of the accompanying drawings which are not intended to limit the scope of the claimed invention:
**FIGURE 1** shows a schematic plan view of a first embodiment of a fluidic network for use in the method of the invention;
**FIGURE 2** shows a schematic cross-sectional view of a second embodiment of a fluidic network for use in the invention; and
**FIGURE 3** shows a schematic cross-sectional view of a third embodiment of a fluidic network for use in the invention.

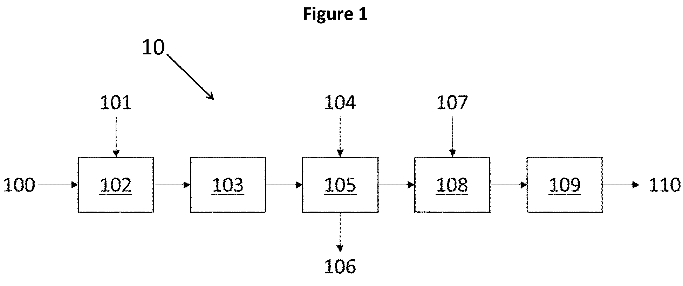

A first embodiment of a fluidic network for use in the method of the invention is indicated generally at 10 on Figure 1. Fluidic network 10 comprises five fluidic modules 102,103,105,108,109. Fluidic modules 102,103,105,108,109 are connected in series. The first fluidic module 102 of fluidic network 10 has two input connectors 100,101.

The fluidic reactor network (10) comprises a first module (102) and a second module (103) fluidically connected to the first module, and a third module (105) fluidically connected to the second module in series, and a fourth module (108) fluidically connected to the third module in series, and a fifth module (109) fluidically connected to the fourth module in series. Additional fluidic modules may be connected in series to include additional unit operations such as in-line purification, in-line analysis or chemical transformation. In some embodiments, in-line analysis includes in-line spectroscopic methods, for example in-line IR. In some embodiments, the fluidic reactor network may be duplicated and run in parallel to increase productivity. In some embodiments, the internal diameter of the fluidic reactor network can be significantly increased for for increasing the productivity.

The first fluidic module 102 is dedicated to mixing two fluids which are input into module 102 by input connectors 100,101 where the two fluids contain at least an organic base and a tosylhydrazone precursor such as the compound of formula (IV). In an alternative embodiment, fluidic module 102 may be composed of embedded mixers or independent mixing elements, such as arrow-head mixers, T-mixers, Y-mixers, cross-junctions or static micromixers, made either of glass, stainless steel, polymeric materials and ceramics, or a combination of two or several of the aforementioned materials.

In an alternative embodiment, fluidic module 102 is not utilized in the fluidic reactor network (10) and the fluid that contains at least an organic base and a tosylhydrazone precursor such as a compound of formula (IV) is directly reacted within the channels of the fluidic module 103.

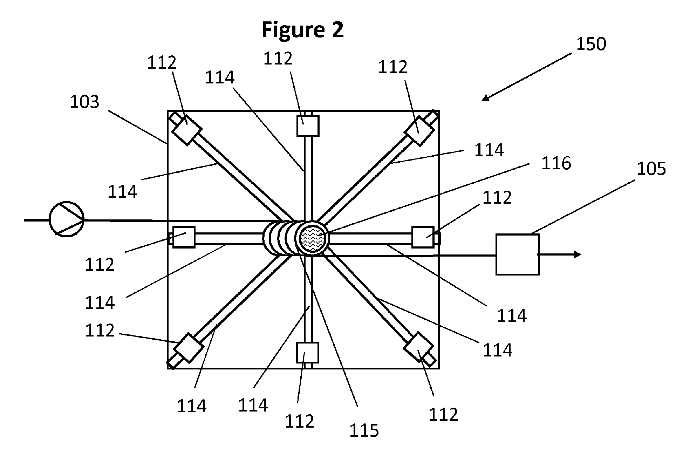

The second fluidic module 103 has an integrated heat exchanger which may be operated at a temperature ranging from -10 °C to 200 °C. In an alternative embodiment, a thermostat or a cryostat or any thermoregulatory device may be utilized to control the temperature of fluidic module 103. In an alternative embodiment, fluidic module 103 may have more than one section such that some sections of fluidic module 103 may be operated at different temperatures at the same time, for example at temperature above 100 °C for a first section of fluidic module 103, and below 50 °C in a last section of fluidic module 103. In an alternative embodiment, fluidic module 103 may be operated at a pressure ranging from 1 to 30 bar. In an alternative embodiment, fluidic module 103 has an integrated static mixer. In an alternative embodiment, the internal volume of fluidic module 103 may be increased by inserting an additional fluidic element. In an alternative embodiment, fluidic module 103 is constructed from a transparent material enabling the irradiation of the internal volume of fluidic module 103. In an alternative embodiment, fluidic module 103 has an integrated light source such as a fluorescent light source, Mercury Vapor light or LED with appropriate wavelengths ranging from 275 to 450 nm, for example from 350 to 430 nm, and specifically from 365 to 405 nm. In an alternative embodiment, fluidic module 103 may be as illustrated in Figure 2 for the second embodiment of the fluidic network 150 which is composed of four distinct elements: (a) 8 adjustable heat-sink integrated pillars 112 each supporting high power LEDs, (b) a refrigerated central cylinder 116, (c) the actual microfluidic reactor 115 made of a PFA coil wrapped around the central cylinder 116 and (d) a docking station with 8 guide rails 114; where the LEDs are mounted to face towards the PFA coil 115 wrapped around the central cylinder 116.

The third fluidic module 105 is composed of an inlet for an extraction fluid 104, a mixing area, a separation device, an outlet for a waste stream and a back-pressure regulator on each outlet set wherein the back-pressure regulators have a cracking pressure ranging from 1 to 30 bar, for example from 5 to 15 bar. In an alternative embodiment, the extraction fluid 104 is a secondary phase, such as an aqueous phase. In an alternative embodiment, the aqueous phase contains an inorganic salt such as sodium chloride. In an alternative embodiment, the secondary aqueous phase is utilized to remove at least one given side-product or impurity from the upstream process. The mixing area may be composed of mixing elements such as arrow-head mixers, T-mixers, Y-mixers, cross-junctions or static micromixers, made either of glass, stainless steel, polymeric materials and ceramics, or a combination of two or several of the aforementioned materials. The mixing area may also be composed of a packed-bed reactor packed with beads made of an inert material such as glass, stainless steel or ceramic with a diameter ranging from 0.05 to 1 mm, but preferably from 0.1 to 0.2 mm. In an alternative embodiment, fluidic module 105 is composed of one or more elements for performing quench, liquid-liquid extraction, liquid-liquid separation, gas-liquid separation, filtration on silica gel or recrystallization, such as, but not restricted to, an in-line liquid-liquid or gas-liquid separation device involving a membrane or a settling tank. In an alternative embodiment, the outlet 106 is redirected to a waste tank which contains an aqueous fluid. In an alternative embodiment, the outlet 106 is redirected to a waste tank which contains an aqueous fluid and a gas. In an alternative embodiment, in-line analysis including, but not restricted to, in-line IR monitoring or other spectroscopic methods, may be integrated in fluidic module 105. In an alternative embodiment, fluidic module 105 only regulates the upstream pressure, ranging from 1 to 30 bar, for example from 5 to 15 bar.

The fourth fluidic module 108 is dedicated to adding fluid 107 to the main organic effluent from upstream fluidic module 105, and to mixing these two fluids that contain at least a pharmaceutically active species precursor such as β-lactam of formula (VI). Fluid 107 may comprise an organic solvent and hydrochloric acid. In an alternative embodiment, fluidic module 108 may comprise an embedded mixer or an inserted independent fluidic element, such as an arrow-head mixer, T-mixer, Y-mixer, cross-junction or static micromixer, made of glass, stainless steel, polymeric material and/or ceramic. In an alternative embodiment, fluidic module 108 is integrated with a heat exchanger, and may be operated at a temperature ranging from 20 to 150 °C, for example from 80 to 120 °C. In an alternative embodiment, a thermostat or a cryostat or other thermoregulatory device may be utilized to control the temperature of fluidic module 108. In an alternative embodiment, fluidic module 108 may comprise two or more sections such that one or more sections of fluidic module 108 may each be operated at different temperatures at the same time, for example at a temperature above 100 °C for the first section of fluidic module 108, and at a temperature below 50 °C in the last section of fluidic element 108. In an alternative embodiment, fluidic module 108 has integrated static mixers. In an alternative embodiment, the internal volume of fluidic module 108 may be increased by inserting one or more additional fluidic elements. In an alternative embodiment, fluidic module 109 may comprises an integrated back-pressure regulator having a cracking pressure setting ranging from 1 to 30 bar, for example from 4 and 10 bar. In an alternative embodiment, fluidic module 108 can be disconnected from fluidic module 105, and the corresponding processes may be run independently.

The fifth fluidic module 109 integrates additional downstream operations such as liquid-liquid extraction, liquid-liquid separation, liquid-solid separation, crystallization, mixing with another fluid containing at least one solvent, formulation, temperature control such as but not restricted to cooling, crystallization, automated collection and/or in-line analysis. In an alternative embodiment, fluidic module 109 can be disconnected from fluidic module 108, and the corresponding processes may be run independently. Fluid 110 comprises an organic solvent, and/or a pharmaceutically active species precursor in solution and/or a pharmaceutically active species in solution and/or a formulation of pharmaceutically active species in solution. In an alternative embodiment, fluid 110 comprises a carrier fluid comprising an organic solvent, and/or organic impurities in solution, and/or a pharmaceutically active species precursor in suspension and/or a pharmaceutically active species in suspension and/or a formulation of pharmaceutically active species in suspension.

A fluidic network according to a second embodiment of the invention for use in the method of the invention is indicated generally at 150 on Figure 2 of the accompanying drawings. Fluidic network 150 comprises a second fluidic module 103, a third fluidic module 105 connected in series. Second fluidic module 103 forms a docking station with eight guide rails 114 arranged symmetrically around a central hub 115,116. The central hub 115,116 comprises a reactor having a 1/16" PFA coil (0.5 mL internal volume, 800 µm internal diameter) 115 which has a central column heat exchanger 116 for heating/or cooling the coil which may be operated at a temperature ranging from -10 °C to 200 °C. Adjustably mounted on each guide rail 114 is a heat sink pillar 112 which supports three high power LEDs where the LEDs are mounted to face towards the PFA coil reactor 115. In an alternative embodiment, the LEDs can be integrated on different supports and according to various configurations to optimize irradiation. Third fluidic module 105 comprises a 13 bar back pressure regulator. In an alternative embodiment, the fluidic network 150 may omit the docking station, guide rails 114,heat sink pillar 112 and the LEDs where irradiation of the coil reactor 115 is not required.

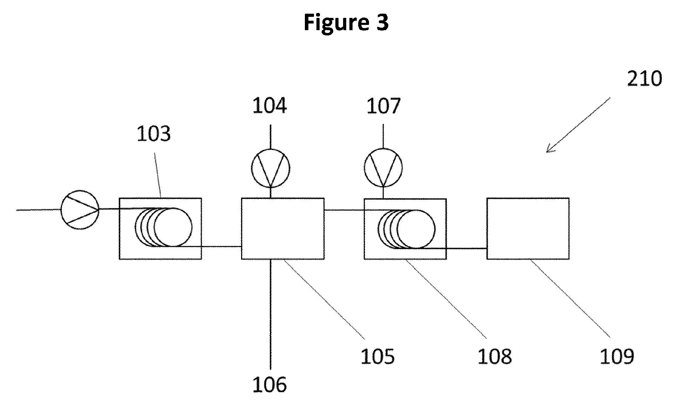

A fluidic network according to a third embodiment of the invention for use in the method of the invention is indicated generally at 210 on Figure 3 of the accompanying drawings. Fluidic network 210 comprises a second fluidic module 103, a third fluidic module 105 connected in series, a fourth fluidic module connected in series and a fifth fluidic module connected in series. Second fluidic module 103 comprises a reactor having a 1/16" PFA coil (0.5 mL internal volume, 800 µm internal diameter) 115 which has a heat exchanger (not shown) for heating/or cooling the coil and/or a light source (not shown) for irradiating the coil. Third fluidic module 105 is composed of an inlet for an extraction fluid 104, a mixing area, a separation device, an outlet 106 for a waste stream and a back-pressure regulator set at a boundary pressure for optimal phase separation. Fourth fluidic module 108 comprises a reactor having a 1/8" PFA coil (6 mL internal volume, 1600 µm internal diameter) which has a heat exchanger (not shown) for heating the coil, a mixer for mixing fluid 107 to organic effluent from upstream fluidic module 105, and a heat exchanger (not shown) to cool the end of the coil. Fifth fluidic module 109 is composed of a back-pressure regulator.

The invention will now be illustrated by reference to the following Examples which are not intended to limit the scope of the invention claimed.

### EXAMPLES

### Example 1. Synthesis of Phenyl-2-(piperidin-1-yl)ethane-1,2-dione (compound of formula (3)).

500 g (3.05 mol, 1 eq.) of methyl benzoylformate (compound of formula **(2)** and 259.7 g (3.05 mol, 1 eq.) of piperidine were vigorously stirred in a 2 L batch glass reactor at 105 °C for 8 h. The reaction mixture was then cooled to room temperature, triturated in petroleum spirit 40-60 and filtered to give 563 g (85%) of compound of formula (3) as an off-white solid.

¹H NMR (CDCl₃, 400 MHz): *δ* = 7.92-7.98 (m, 2H), 7.61-7.67 (m, 1H), 7.51 (t, 2H, J = 7.7 Hz), 3.66-3.75 (m, 2H), 3.25-3.33 (m, 2H), 1.66-1.74 (m, 4H), 1.52-1.56 (m, 2H) ppm; ¹³C NMR (CDCl₃, 100.6 MHz): *δ* = 191.9, 165.4, 134.6, 133.2, 129.5, 129.0, 47.0, 42.1, 26.1, 25.4, 24.3 ppm. ESI-HRMS: [M+H]⁺ calcd: 218.1176, found: 218.1178. MP: 104.7-106.3 °C.

### Example 2. Synthesis of 4-Methyl-N'-(2-oxo-1-phenyl-2-(piperidin-1-yl)ethylidene)benzenesulfono-hydrazide (compound of formula (4))

500 g (2.03 mol, 1 eq.) of α-ketoamide **3,** 415.3 g (2.23 mol, 1.1 eq.) of p-toluenesulfonyl hydrazide and 22.8 g (0.12 mol, 0.06 eq.) of p-toluenesulfonic acid monohydrate were stirred in acetonitrile (5 L) for 15 h at 90 °C. The solvent was evaporated and could be recycled after distillation for other batches. The resulting white solid was washed with diethyl ether and recrystallized from boiling ethanol (3 L/100 g). After filtration, the white needles were washed with diethyl ether to give 626 g (80%) of pure tosylhydrazone (compound of formula **(4).** The mother liquor was distilled and ethanol could be recycled for other recrystallizations.

¹H NMR (DMSO d-6, 400 MHz): *δ* = 11.25 (s, 1H), 7.82 (d, 2H, J = 8.3 Hz), 7.47-7.51 (m, 2H), 7.40-7.46 (m, 5H), 3.58 (brs, 2H), 3.00 (t, 2H, J = 5.5 Hz), 2.37 (s, 3H), 1.57 (brs, 4H), 1.30 (brs, 2H) ppm; ¹³C NMR (DMSO d-6, 100.6 MHz): *δ* = 161.5, 149.1, 143.7, 136.3, 132.9, 130.5, 129.8, 129.1, 127.6, 126.0, 46.2, 41.5, 25.8, 24.9, 23.9, 21.2 ppm. ESI-HRMS: [M+H]⁺ calcd: 386.1533, found: 386.1531. IR (neat): *ν*ₘₐₓ 3009, 2945, 2860, 2792, 1619 cm⁻¹. MP: 190.7-192.6°C

### Example 3. Continuous-flow thermolysis of the compound of formula (4) towards 7-phenyl-1-aza-bicyclo[4.2.0]octan-8-one (compound of formula (6)) on microscale at 150 °C.

In this example, the alternative embodiment of the fluidic network reactor (150) as illustrated in Figure 2 was used which omits omit the docking station, guide rails 114 and heat sink pillar 112 and the LEDs, and which includes an integrated heat exchanger. Toluene (5 mL) was added to 2.89 g (7.50 mmol, 1 eq.) of tosylhydrazone of formula **(4)** in a 10 mL volumetric flask. Then, DBU (1.25 g, 8.21 mmol, 1.1 eq.) was added, the volumetric flask was filled with toluene and the solid was dissolved under vigorous stirring and sonication. The solution was then loaded into a syringe, and injected into the second module 103 of the microfluidic network reactor 150 at appropriate flow rate (0.1 mL min⁻¹). The reactor of the second module 103 was heated at 150 °C. The reactor reached steady state after 1.5 residence times (7.5 min), and the effluent of the reactor was collected and then analyzed by HPLC and by ¹ H NMR in CDCl₃ on the resulting crude material. Analyses revealed 99% conversion. Filtration on silica gel with 6:4 petroleum ether/ethyl acetate gave essentially pure 7-phenyl-1-aza-bicyclo[4.2.0]octan-8-one **(6).** The results for Example 3 are shown in Table 1 below where Vᵢₙₜ represents the internal volume of the reactor 150.

**TABLE 1: Results for Example 3**

| **Entry** | **Vᵢₙₜ (mL)** | **Flow (mL min⁻¹)** | **Conversion (%)** | **Threo/Erythro** |
|---|---|---|---|---|
| 1 | 0.5 | 0.1 | >99 | 3:1 |
| 2 | 0.5 | 0.05 | >99 | 4.2:1 |

### Example 4: Continuous-flow thermolysis of the compound of formula (4) towards 7-phenyl-1-aza-bicyclo[4.2.0]octan-8-one of formula (6) on microscale at 180 °C

In this example, the alternative embodiment of the fluidic reactor network (150) as illustrated in Figure 2 was used which omits omit the docking station, guide rails 114 and heat sink pillar 112 and the LEDs, and which includes an integrated heat exchanger. Toluene (5 mL) was added to 2.89 g (7.50 mmol, 1 eq.) of tosylhydrazone **4** in a 10 mL volumetric flask. Then, DBU (1.25 g, 8.21 mmol, 1.1 eq.) was added, the volumetric flask was filled with toluene, and the solid was dissolved under vigorous stirring and sonication. The solution was then loaded into a syringe, and injected into the second module 103 of microfluidic network reactor 150 at appropriate flow rate (0.1 mL min⁻¹) and the reactor was heated at 180 °C. The reactor reached steady state after 1.5 residence times (7.5 min), and the effluent of the reactor was collected and then analyzed by HPLC and by ¹ H NMR in CDCl₃ on the resulting crude material. Analyses revealed 99% conversion and a 4.2:1 threo/erythro ratio. Filtration on silica gel with 6:4 petroleum ether/ethyl acetate gave essentially pure 7-phenyl-1-aza-bicyclo[4.2.0]octan-8-one of formula **(6).**

**TABLE 2: Results of Example 4**

| **Entry** | **Vᵢₙₜ (mL)** | **Flow (mL min⁻¹)** | **Conversion (%)** | **Threo/Erythro** |
|---|---|---|---|---|
| 1 | 0.5 | 0.1 | >99 | 4:1 |

### Example 5: Continuous-flow thermolysis of a compound of formula (4) towards 7-phenyl-1-aza-bicyclo[4.2.0]octan-8-one a compound of formula (6) in the presence of Aliquat^{®} 336 on microscale at 150 °C

In this example, the alternative embodiment of fluidic reactor network (150) as illustrated in Figure 2 was used which omits omit the docking station, guide rails 114 and heat sink pillar 112, and the LEDs, and which includes an integrated heat exchanger. Toluene (5 mL) was added to 2.89 g (7.50 mmol, 1 eq.) of tosylhydrazone of formula **(4)** and to 0.332 g of Aliquat^{®} 336 (0.75 mmol, 0.1eq.) in a 10 mL volumetric flask. Then, DBU (1.25 g, 8.21 mmol, 1.1 eq.) was added, the volumetric flask was filled with toluene, and the solid was dissolved under vigorous stirring and sonication. The solution was then loaded into a syringe, and injected into the second fluidic module 103 of fluidic reactor network 150 at appropriate flow rate (0.05 mL min⁻¹) and the reactor was heated at 150 °C. The reactor reached steady state after 1.5 residence times (15 min), and the effluent of the reactor was collected and then analyzed by HPLC and by ¹ H NMR in CDCl₃ on the resulting crude material. Analyses revealed 99% conversion and a 4.2:1 threo/erythro ratio. Filtration on silica gel with 6:4 petroleum ether/ethyl acetate gave essentially pure 7-phenyl-1-aza-bicyclo[4.2.0]octan-8-one of formula **(6).**

**TABLE 3: Results of Example 5**

| **Entry** | **Vᵢₙₜ (mL)** | **Flow (mL min⁻¹)** | **Conversion (%)** | **Threo/Erythro** |
|---|---|---|---|---|
| 1 | 0.5 | 0.1 | 79 | 4.1:1 |
| 2 | 0.5 | 0.05 | >99 | 4.2:1 |

### Example 6: Continuous-flow thermolysis of a compound of formula (4) towards 7-phenyl-1-aza-bicyclo[4.2.0]octan-8-one of formula (6) in the presence of Aliquat^{®} 336 on microscale at 180 °C.

In this example, the alternative embodiment of the fluidic reactor network (150) as illustrated in Figure 2 was used which omits omit the docking station, guide rails 114 and heat sink pillar 112 and the LEDs, and which includes an integrated heat exchanger. Toluene (5 mL) was added to 2.89 g (7.50 mmol, 1 eq.) of tosylhydrazone of formula **(4)** and to 0.332 g of Aliquat^{®} 336 (0.75 mmol, 0.1eq.) in a 10 mL volumetric flask. Then, DBU (1.25 g, 8.21 mmol, 1.1 eq.) was added, the volumetric flask was filled with toluene, and the solid was dissolved under vigorous stirring and sonication. The solution was then loaded into a syringe, and injected into the microfluidic reactor network 150 at appropriate flow rate (0.1 mL min⁻¹) and the reactor was heated at 180 °C. The reactor reached steady state after 1.5 residence times (7.5 min), and the effluent of the reactor was collected and then analyzed by HPLC and by ¹H NMR in CDCl₃ on the resulting crude material. Analyses revealed 99% conversion and a 4:1 threo/erythro ratio. Filtration on silica gel with 6:4 petroleum ether/ethyl acetate gave essentially pure 7-phenyl-1-aza-bicyclo[4.2.0]octan-8-one of formula **(6).**

**TABLE 4: Results of Example 6**

| **Entry** | **Vᵢₙₜ (mL)** | **Flow (mL min⁻¹)** | **Conversion (%)** | **Threo/Erythro** |
|---|---|---|---|---|
| 1 | 0.5 | 0.1 | >99 | 4:1 |

### Example 7: Continuous-flow photolysis of the compound of formula (4) towards 7-phenyl-1-aza-bicyclo[4.2.0]octan-8-one of formula (6) in the presence of Aliquat^{®} 336 on microscale with 24 LEDs@395 nm.

In this example, the fluidic reactor network (150) as illustrated in Figure 2 was used. Toluene (5 mL) was added to 2.89 g (7.50 mmol, 1 eq.) of tosylhydrazone of formula **(4)** and to 0.332 g of Aliquat^{®} 336 (0.75 mmol, 0.1eq.) in a 10 mL volumetric flask. Then, DBU (1.25 g, 8.21 mmol, 1.1 eq.) was added, the volumetric flask was filled with toluene, and the solid was dissolved under vigorous stirring and sonication. The solution was then loaded into a syringe, and injected into the second fluidic module 103 of microfluidic reactor network 150 at appropriate flow rate (0.033 mL min⁻¹) towards a reactor irradiated at 395 nm at 45 °C. The reactor reached steady state after 1.5 residence times (90 min), and the effluent of the reactor was collected and then analyzed by HPLC and by ¹ H NMR in CDCl₃ on the resulting crude material. Analyses revealed 97% conversion and a 3.9:1 threo/erythro ratio.

**TABLE 5: Results of Example 7**

| **Entry** | **Vᵢₙₜ (mL)** | **Flow (mL min⁻¹)** | **Conversion (%)** | **Threo/Erythro** |
|---|---|---|---|---|
| 1 | 2 | 0.044 | 62 | 5.1:1 |
| 2 | 2 | 0.033 | 97 | 3.9:1 |
| 3 | 3 | 0.05 | 76 | 3.8:1 |

### Example 8. In-line methanolysis of 7-phenyl-1-aza-bicyclo[4.2.0]octan-8-one of formula (6) towards methylphenidate hydrochloride of formula (1)·HCl.

In this example, the fluidic reactor network (210) as illustrated in Figure 3 was used which comprises a first module (103) and a second module (105) fluidically connected to the first module, and a third module (108) fluidically connected to the second module in series, and a fourth module (109) fluidically connected to the third module in series. Toluene (15 mL) was added to 7.23 g (18.75 mmol, 1 eq.) of tosylhydrazone of formula **(4)** and to 0.83 g of Aliquat^{®} 336 (1.88 mmol, 0.1 eq.) in a 25 mL volumetric flask. Then, DBU (3.13 g, 20.53 mmol, 1.1 eq.) was added, the volumetric flask was filled with toluene, and the solid was dissolved under vigorous stirring and sonication. The solution was then loaded into a syringe and injected at 0.1 mL min⁻¹ flow rate towards a microfluidic reactor constructed from a 1/16" PFA coil (0.5 mL internal volume, 800 µm internal diameter) heated at 150 °C. A 10 wt% NaCl aqueous solution was prepared by dissolving 2 g of sodium chloride into 18 g of deionized water. It was loaded into a syringe and injected at a flow rate of 0.1 mL min⁻¹ into the system described in Figure 3. The subsequent biphasic mixture was next sent to a packed bed column (10 cm of 1/4" PFA tubing filled with 0.1 mm glass beads), before entering a low dead volume membrane separator (A. Adamo et al., 2013, Ind. Eng. Chem. Res. 52:10802-10808). Two back pressure regulators were inserted on the permeate and the retentate outlet, respectively, and both were set at 13 bar. An acidic methanol solution was prepared by mixing 5 mL of HCl 4 M in dioxane with 15 mL of methanol. It was then loaded into a syringe, injected at a 0.2 mL min⁻¹ flow rate and mixed with the organic permeate towards a microfluidic reactor according to a third embodiment of the invention constructed from a 1/8" PFA coil (6 mL internal volume, 800 µm internal diameter) heated at 110 °C (Figure 3, Scheme 2 and Table 6). A back pressure regulator set at 5 bar and heated at 80 °C was inserted downstream. An appropriate combination of solvents induced precipitation of methylphenidate hydrochloride as a white solid in the collection flask. The effluent of the reactor was collected and analyzed. Enriched threo methylphenidate hydrochloride was obtained in 70% overall yield. Further epimerization or selective crystallization gave essentially pure threo methylphenidate hydrochloride.

**TABLE 5: Results of Example 8**

| **Entry** | **T (°C)** | **t (min) in µFR#2** | **HCl (M)** | **HCl/6** | **Conversion (%)** |
|---|---|---|---|---|---|
| 1 | rt | 5 | 0.5 M^{a} | 1:0.75 | 35 |
| 2 | 50 | 5 | 0.5 M^{b} | 1:0.75 | 47 |
| 3 | 80 | 5 | 0.5 M^{b} | 1:0.75 | 56 |
| 4 | 80 | 10 | 0.5 M^{b} | 1:0.75 | 57 |
| 5 | 100 | 10 | 1 M^{c} | 2:0.75 | 61 |
| 6 | 100 | 20 | 1 M^{c} | 2:0.75 | 81 |
| 7 | 110 | 20 | 1 M^{c} | 2:0.75 | >99 |

| | | | | | |
|---|---|---|---|---|---|
| ^{a} MeOH/Et₂O 1:1; ^{b} MeOH/Et₂O 3:1; ^{c} MeOH/dioxan 3:1 | | | | | |

Characterization of the racemate of threo methylphenidate hydrochloride: ¹H NMR (D₂O, 400 MHz): *δ* = 7.30 (d, 3H, J = 6.6 Hz), 7.17 (d, 2H, J = 6.3 Hz), 3.86 (d, 1H, J = 9.2 Hz), 3.68 (t, 1H, J = 10.1 Hz), 3.57 (s, 3H), 3.32 (d, 1H, J = 12.7 Hz), 2.94 (t, 1H, J = 12.8 Hz), 1.72 (d, 1H, J = 14.3 Hz), 1.63 (d, 1H, J = 11.4 Hz), 1.39-1.54 (m, 2H), 1.16-1.35 (m, 2H) ppm.¹³C NMR (D₂O, 100.6 MHz): *δ* = 173.2, 133.3, 129.5, 128.9, 128.6, 57.8, 53.7, 53.2, 45.6, 26.2, 21.8, 21.3 ppm. ESI HRMS: [M+H]⁺ calcd: 234.1489, found: 234.1488. IR (neat): *ν*ₘₐₓ 2934, 2804, 2703, 2512, 2413, 1738 cm⁻¹. MP: 218.4-219.8 °C.

Chiral HPLC analysis of a sample of the racemate of threo methylphenidate hydrochloride illustrates the elution of both threo diastereoisomers perfectly separated (7.665 and 9.162 min) (not shown).

### Example 9: Continuous-flow thermolysis of the compound of formula (4) towards 7-phenyl-1-aza-bicyclo[4.2.0]octan-8-one of formula (6) in the presence of Aliquat^{®} 336 on meso-scale at 180 °C

In this example, the alternative embodiment of the fluidic reactor network (150) as illustrated in Figure 2 was used which omits omit the docking station, guide rails 114 and heat sink pillar 112, and the LEDs, and which includes an integrated heat exchanger. In a typical procedure, toluene (25 mL) was added to 14.45 g (37.5 mmol, 1 eq.) of tosylhydrazone of formula **(4)** and to 1.66 g of Aliquat^{®} 336 (3.76 mmol, 0.1 eq.) in a 50 mL volumetric flask. Then, DBU (6.25 g, 41.1 mmol, 1.1 eq.) and toluene were added, and the solid was dissolved under vigorous stirring and sonication. The solution was injected at a 0.4 mL min⁻¹ flow rate towards a continuous meso-fluidic reactor made of specialty glass (Corning^{®} Advanced-Flow™ LowFlow reactor, 2 mL total internal volume) integrated with a heat-exchanger operated at 180 °C. The reactor effluent was cooled down before the back pressure regulator set at 5 bar and inserted downstream. Steady state was reached after 1.5 residence time (5 min) and the effluent of the reactor was collected and analyzed.

### Example 10: Continuous-flow thermolysis of the compound of formula (4) towards 7-phenyl-1-aza-bicyclo[4.2.0]octan-8-one of formula (6) in the presence of Aliquat^{®} 336 on pilot scale at 180 °C

In this example, the alternative embodiment of the fluidic reactor network (150) as illustrated in Figure 2 was used which omits omit the docking station, guide rails 114 and heat sink pillar 112 and the LEDs, and which includes an integrated heat exchanger. Toluene (500 mL) was added to a mixture of 289 g (0.75 mol, 1 eq.) of tosylhydrazone of formula **(4)** and to 49.8 g (0.113 mol, 0.15 eq.) of Aliquat^{®} 336 in a 1 L volumetric flask. Then, DBU (125 g, 0.821 mol, 1.1 eq.) and toluene were added, and the solid was dissolved under vigorous stirring and sonication. The solution was injected at a 20 g min⁻¹ flow rate towards a continuous meso-fluidic pilot scale reactor 150 made of specialty glass (Corning^{®} Advanced-Flow™ G1 reactor, 36 mL total internal volume) integrated with a heat-exchanger operated at 180 °C. The reactor effluent was cooled down before the back pressure regulator set at 5 bar and inserted downstream. Steady state was reached after 1.5 residence time (2.7 min) and the effluent of the reactor was collected and analyzed. Analyses revealed 99% conversion and a 3:1 threo/erythro ratio.

¹H NMR (CDCl₃, 400 MHz): *δ* = 7.21-7.38 (m, 5H), 4.61 (d, 0.25H, J = 4.6 Hz), 3.87-3.99 (m, 1.75H), 3.68 (m, 0.25H), 3.37 (dd, 0.75H, J = 9.1, 4.8 Hz), 2.70-2.86 (m, 1H), 2.18 (dt, 0.75H, J = 9.1, 4.7 Hz), 1.92 (brs, 0.75H), 1.80 (d, 0.25H, J = 11.5 Hz), 1.60-1.75 (m, 1H), 1.30-1.53 (m, 3H), 0.81-0.95 (m, 0.25H) ppm. ¹³C NMR (CDCl₃, 100.6 MHz): *δ* = 166.6, 166.2, 135.6, 133.5, 128.6, 128.4, 128.3, 127.2, 127.0, 63.4, 58.2, 56.7, 52.6, 38.9, 38.6, 30.4, 26.7, 24.8, 24.4, 22.1, 21.7 ppm. ESI-HRMS: [M+H]⁺ calcd: 202.1226, found: 202.1227. IR (neat): *ν*ₘₐₓ 2943, 2896, 2858, 1728 cm⁻¹. MP: 65.4-68.8 °C

### Example 11: Continuous-flow photolysis of the compound of formula (4) towards 7-phenyl-1-aza-bicyclo[4.2.0]octan-8-one of formula (6) in the presence of Aliquat^{®} 336 on pilot scale at 30 °C

In this example, the fluidic reactor network (150) as illustrated in Figure 2 was used. Toluene (500 mL) was added to a mixture of 28.9 g (0.075 mol, 1 eq.) of tosylhydrazone of formula **(4)** and to 4.98 g (0.011 mol, 0.15 eq.) of Aliquat^{®} 336 in a 1 L volumetric flask. Then, DBU (1.25 g, 0.082 mol, 1.1 eq.) and toluene were added, and the solid was dissolved under vigorous stirring and sonication. The solution was injected at a 10 g min⁻¹ flow rate towards a continuous meso-fluidic pilot scale reactor 150 made of specialty glass (Corning^{®} Advanced-Flow™ G1 Photoreactor, 36 mL total internal volume) integrated with a heat-exchanger operated at 30 °C and integrated with LED panels (300 LEDs in total). The reactor effluent was cooled down before the back pressure regulator set at 5 bar and inserted downstream. Steady state was reached after 1.5 residence time (5 min) and the effluent of the reactor was collected and analyzed. Analyses revealed 74% conversion and a 4:1 threo/erythro ratio.

## Claims

1. A method for the preparation of an intermediate for use in synthesizing a lower alkyl phenidate compound of formula (I): 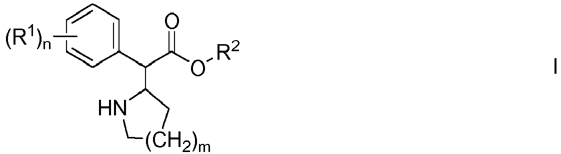 wherein each R¹ independently represents an optionally substituted aryl, heteroaryl, alkyl, perfluoroalkyl, cycloalkyl, alkoxy, aryloxy, acyl, carboxyl, hydroxyl, halogen, amino, nitro, cyano, sulfo or sulfhydryl group, R² represents a hydrogen atom or a lower alkyl group, n represents an integer from 1 to 5 and m represents an integer from 1 to 3 or a pharmaceutically acceptable salt thereof; which method comprises the steps of:
(a) flowing a tosylhydrazone compound of formula (IV): 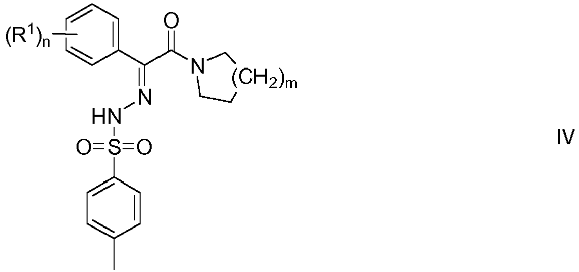 wherein R¹, n and m are as defined above in relation to the methylphenidate of formula (I), an organic base and an organic solvent into a fluidic network; and
(b) reacting the tosylhydrazone compound of formula (IV) and the base in the fluidic network under thermal and/or photochemical conditions to form a transient diazoamide compound of formula (V): 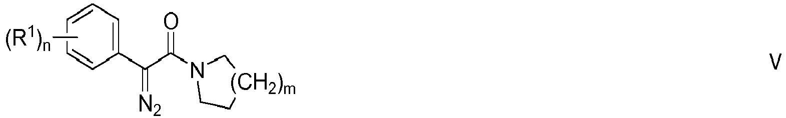 wherein R¹, n and m are as defined above in relation to the methylphenidate of formula (I).

2. A method as defined in Claim 1 which additionally comprises the following step:
(c) thermal and/or photochemical decomposition of the diazoamide compound of formula (V) in a fluidic network to yield a pharmaceutically active species precursor such as a β-lactam of formula (VI): 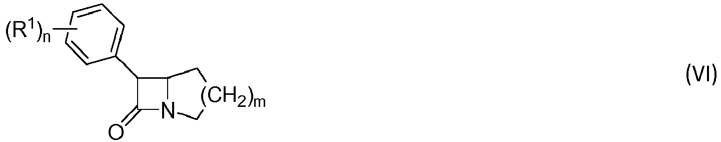 wherein R¹, m and n are as defined in Claim 1.

3. A method as defined in Claim 2 wherein step (c) further comprises one of the following reactions:
(i) a reaction of the β-lactam of formula (VI) with a lower alkyl alcohol in the presence of a pharmaceutically acceptable acid to form a lower alkyl phenidate of formula (I) or a salt or an analog thereof; or
(ii) a reaction with an organic and/or inorganic base or acid to increase the amount of a specific stereoisomer of the compound of formula (VI); or
(iii) a reaction with an organic and/or inorganic base or acids to increase the amount of a specific stereoisomer of the compound of formula (I).

4. A method as defined in Claim 3 wherein step (c)(i) is performed for a reaction time of about 30 min or less.

5. A method as defined in Claim 3 wherein step (c)(ii) or (c)(iii) is performed for a reaction time of about 60 min or less

6. A method as defined in any one of the preceding Claims wherein the organic base is a nitrogenous organic base.

7. A method as defined in any one of the preceding Claims which uses in step (b) an aprotic polar solvent or a non-water soluble apolar aprotic organic solvent.

8. A method as defined in any one of the preceding Claims wherein the phase transfer catalyst improves the solubility of the tosylhydrazone of formula (IV); preferably the phase transfer catalyst is a quaternary ammonium salt.

9. A method as defined in any one of the preceding Claims wherein the fluidic network is a micro-fluidic network.

10. A method as defined in any one of the preceding Claims wherein the fluidic network is a meso-fluidic network.

11. A method as defined in any one of the preceding Claims wherein the fluidic network comprises one or more fluidic modules connected in series.

12. A method as defined in any one of the preceding Claims wherein two or more fluidic networks are operated in parallel; preferably the two or more fluidic networks are substantially identical.

13. A method as defined in any one of the preceding Claims wherein step (a) includes flowing a phase transfer catalyst into the fluidic network.

14. A method as defined in claim 13 wherein the phase transfer catalyst is a quaternary ammonium salt.
